(19) **Europäisches Patentamt / European Patent Office / Office européen des brevets**

(11) **EP 3 744 826 A1**

(12) # EUROPEAN PATENT APPLICATION
published in accordance with Art. 153(4) EPC

(43) Date of publication:
**02.12.2020 Bulletin 2020/49**

(51) Int Cl.:
**C12M 3/00** (2006.01)        **C12M 1/00** (2006.01)
**C12N 5/071** (2010.01)

(21) Application number: **19744054.8**

(22) Date of filing: **24.01.2019**

(86) International application number:
**PCT/JP2019/002375**

(87) International publication number:
**WO 2019/146732 (01.08.2019 Gazette 2019/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2018 JP 2018010105**

(71) Applicant: Ube Industries, Ltd.
**Yamaguchi 755-8633 (JP)**

(72) Inventors:
- **HAGIHARA, Masahiko**
  **Ube-shi, Yamaguchi 755-8633 (JP)**
- **HARADA, Takashi**
  **Ube-shi, Yamaguchi 755-8633 (JP)**
- **MATSUBAYASHI, Akihiro**
  **Ichihara-shi, Chiba 290-0045 (JP)**
- **FUSE, Shinsaku**
  **Ube-shi, Yamaguchi 755-8633 (JP)**

(74) Representative: Plougmann Vingtoft a/s
**Strandvejen 70**
**2900 Hellerup (DK)**

(54) **CELL CULTURE MODULE**

(57) Provided is a means that applies uniform culture conditions to a plurality of porous polymer membranes and makes it possible to stably culture a large quantity of cells. A cell culture module that comprises: a top part; a bottom part; a side part that has a culture solution in/outflow port; a plurality of partition parts that partition a space that is formed by the top part, the bottom part, and the side part and have a culture solution in/outflow port; and porous polymer membranes that are respectively fixed in at least two interstitial spaces selected from the interstitial space between the top part and the adjacent partition part, the interstitial space between the bottom part and the adjacent partition part, and the plurality of interstitial spaces between adjacent partition parts. The porous polymer membranes have a three-layer structure that includes: a surface layer A and a surface layer B that have a plurality of pores; and a macrovoid layer that is sandwiched between surface layer A and surface layer B. The average pore diameter of the pores in surface layer A is smaller than the average pore diameter of the pores in surface layer B. The macrovoid layer has: a diaphragm that is bonded to surface layers A and B; and a plurality of macrovoids that are surrounded by the diaphragm and surface layers A and B. The pores in surface layers A and B communicate with the macrovoids.

EP 3 744 826 A1

**Description**

FIELD

The present invention relates to a cell culture module.

BACKGROUND

In recent years, proteins such as enzymes, hormones, antibodies, cytokines, viruses (viral proteins) used for treatment and vaccine are industrially produced using cultured cells. However, such a protein production technology is expensive, raising medical cost. Accordingly, there have been demands for innovating technologies for culturing cells at high density and for increasing protein production, aiming at great reduction of cost.

As cells for protein production, anchorage-dependent adherent cells which adhere to a culture substrate may be sometimes used. Since such cells grow anchorage-dependently, they need to be cultured while being adhered onto the surface of a dish, plate or chamber. Conventionally, in order to culture such adherent cells in a large amount, it was preferable to increase the surface area to be adhered. However, increasing the culturing area inevitably requires to increase the space, which is responsible for increase in cost.

As a method to culture a large amount of adherent cells while decreasing the culture space, a method for culture using a microporous carrier, especially a microcarrier, has been developed (for example, PTL 1). In a cell culturing system using microcarriers, it is preferable to carry out sufficient stirring and diffusion so that the microcarriers do not aggregate together. Since this requires a volume allowing adequate agitation and diffusion of the medium in which the microcarriers are dispersed, there is an upper limit to the density at which the cells can be cultured. In order to separate the microcarrier from the medium, separation is preferably performed using a filter which can separate fine particles, possibly resulting in increased cost. Considering the foregoing, there is a demand for innovative methodology for cell culture which cultures cells at high density.

< Porous Polyimide Film >

Porous polyimide films have been utilized in the prior art for filters and low permittivity films, and especially for battery-related purposes, such as fuel cell electrolyte membrane and the like. PTLs 2 to 4 describe porous polyimide films with numerous macrovoids, having excellent permeability to objects such as gases, high porosity, excellent smoothness on both surfaces, relatively high strength and, despite high porosity, excellent resistance against compression stress in the film thickness direction. All of these are porous polyimide films formed via amic acid.

The cell culture method which includes applying cells to a porous polyimide film and culturing them is reported (PTL 5).

[CITATION LIST]

[PATENT LITERATURE]

[PTL 1] WO2003/054174
[PTL 2] WO2010/038873
[PTL 3] Japanese Unexamined Patent Publication (Kokai) No. 2011-219585
[PTL 4] Japanese Unexamined Patent Publication (Kokai) No. 2011-219586
[PTL 5] WO2015/012415

SUMMARY

[TECHNICAL PROBLEM]

In the case of shaking culture or stirring culture of cells using a plurality of porous polyimide films in one culture vessel or bag, respective forces received by the porous polyimide films are different in the vessel or bag. Accordingly, it has been difficult to perform cell culture for all the porous polyimide films under homogeneous conditions. In addition, in the case of shaking culture or stirring culture using porous polyimide films, it has been difficult to perform stable cell culture since stress is applied to cells growing in the films due to the continuous deformation of the shapes of the films, and the cells die.

Accordingly, it is an object of the present invention to provide means with which it is possible to apply a homogeneous culture condition to a plurality of porous polymer films and to stably culture a large amount of cells.

[SOLUTION TO PROBLEM]

As a result of intensive examination in light of the problems described above, the present inventors found that it is possible to stably culture a large amount of cells by using a module including a plurality of porous polymer films and having a specific tertiary structure, and thus accomplished the present invention.

The present invention includes the following <1> to <15>.

<1> A cell culture module comprising:

an apex part;
a bottom part;
a side part comprising a culture medium flow inlet;
a plurality of partition parts that partition a space formed by the apex part, the bottom part, and the side part, and comprise a culture medium flow inlet; and
a porous polymer film fixed to each of two or more gap spaces selected from a gap space between the apex part and a partition part adjacent thereto, a gap space between the bottom part and a partition part adjacent thereto, and a plurality of gap spaces between partition parts adjacent to each other,
wherein the porous polymer film is a porous polymer film with a three-layer structure, comprising a surface layer A and a surface layer B including a plurality of pores, as well as a macrovoid layer sandwiched between the surface layer A and the surface layer B, an average pore diameter of the pores present in the surface layer A is smaller than an average pore diameter of the pores present in the surface layer B, the macrovoid layer comprises a partition wall bonded to the surface layers A and B, and a plurality of macrovoids surrounded by the partition wall and the surface layers A and B, and the pores in the surface layers A and B communicate with the macrovoids.

<2> The cell culture module according to <1>, wherein at least one of gap spaces adjacent to the gap space to which the porous polymer film is fixed does not comprise a porous polymer film.
<3> The cell culture module according to <1> or <2>, wherein 3 to 100 porous polymer films are layered and placed on each of the two or more gap spaces selected from the gap space between the apex part and the partition part adjacent thereto, the gap space between the bottom part and the partition part adjacent thereto, and the plurality of gap spaces between the partition parts adjacent to each other.
<4> The cell culture module according to any one of <1> to <3>, wherein the porous polymer film is a porous polyimide film.
<5> The cell culture module according to any one of <1> to <3>, wherein the porous polymer film is a porous polyethersulfone film.
<6> The cell culture module according to any one of <1> to <5>, wherein the apex part and the bottom part comprise a culture medium flow inlet.
<7> The cell culture module according to any one of <1> to <6>, comprising a rectangular parallelepiped shape.
<8> The cell culture module according to any one of <1> to <6>, comprising a cube shape.
<9> The cell culture module according to any one of <1> to <6>, comprising an ovoid shape.
<10> A cell culture module complex, wherein the plurality of cell culture modules according to <7> or <8> are connected.
<11> A cell culture module comprising:

a plurality of cell culture submodules; and
a casing for containing a cell culture submodule, which is used for layering and containing the plurality of cell culture submodules and comprises a culture medium flow inlet,
wherein the cell culture submodules comprise:

a porous polymer film; and
a casing for containing a porous polymer film, comprising a culture medium flow inlet, wherein the porous polymer film is fixed and contained, and
wherein the porous polymer film is a porous polymer film with a three-layer structure, comprising a surface layer A and a surface layer B including a plurality of pores, as well as a macrovoid layer sandwiched between the surface layer A and the surface layer B, an average pore diameter of the pores present in the surface layer A is smaller than an average pore diameter of the pores present in the surface layer B, the macrovoid layer comprises a partition wall bonded to the surface layers A and B, and a plurality of macrovoids surrounded by the partition wall and the surface layers A and B, and the pores in the surface layers A and B communicate with the macrovoids.

<12> The cell culture module according to <11>, further comprising a gap space between the plurality of layered cell culture submodules and the casing for containing a cell culture submodule.

<13> The cell culture module according to <11> or <12>, wherein 3 to 100 porous polymer films are layered and contained in the casing for containing a porous polymer film.

<14> The cell culture module according to any one of <11> to <13>, wherein the porous polymer film is a porous polyimide film.

<15> The cell culture module according to any one of <11> to <13>, wherein the porous polymer film is a porous polyethersulfone film.

[ADVANTAGEOUS EFFECTS OF INVENTION]

In accordance with the present invention, it is possible to apply a homogeneous culture condition to a plurality of porous polymer films, and to stably culture a large amount of cells.

BRIEF DESCRIPTION OF DRAWINGS

FIG. 1 is a perspective view of a cell culture module 1. However, an illustration of porous polymer films in the cell culture module are omitted.

FIG. 2 is a front view for explaining the interior of the cell culture module 1.

FIG. 3 is a perspective view for explaining the interior of the cell culture module 1.

FIG. 4 is a perspective view for explaining the interior of the cell culture module 1, cut along the line A-A in FIG. 1.

FIG. 5 is a perspective view of a cell culture module complex 10 produced by preparing two cell culture modules 1 illustrated in FIGs. 1 to 3 and connecting the apex parts of one cell culture module 1 and the other cell culture module 1 to each other. However, an illustration of porous polymer films in the cell culture modules is omitted.

FIG. 6 is a front view of the cell culture module complex 10.

FIG. 7 is a perspective view of a cell culture module 20.

FIG. 8 is a front view of the cell culture module 20.

FIG. 9 is a top view of a cell culture submodule 30 used in the cell culture module 20 illustrated in FIGs. 7 and 8.

FIG. 10 is a cross-sectional view of the cell culture submodule 30 taken along the line B-B in FIG. 9.

FIG. 11 illustrates variations with time of the amount of antibody produced by cell culture using the cell culture module 20.

DESCRIPTION OF EMBODIMENTS

Embodiments of the present invention will be described below with reference to the drawings as needed. The configurations of the embodiments are illustrative, and the constitution of the present invention is not limited to the specific configurations of the embodiments.

<< Porous Polymer Film >>

An average pore diameter of the pore present on a surface layer A (hereinafter referred to as "surface A" or "mesh surface") in the porous polymer film used for the present invention is not particularly limited, but is, for example, 0.01 $\mu$m or more and less than 200 $\mu$m, 0.01 to 150 $\mu$m, 0.01 to 100 $\mu$m, 0.01 to 50 $\mu$m, 0.01 to 40 $\mu$m, 0.01 to 30 $\mu$m, 0.01 to 25 $\mu$m, 0.01 to 20 $\mu$m, or 0.01 to 15 $\mu$m, preferably 0.01 to 25 $\mu$m.

The average pore diameter of the pore present on a surface layer B (hereinafter referred to as "surface B" or "large pore surface") in the porous polymer film used for the present invention is not particularly limited so long as it is larger than the average pore diameter of the pore present on the surface A, but is, for example, greater than 5 $\mu$m and 200 $\mu$m or less, 20 $\mu$m to 100 $\mu$m, 25 $\mu$m to 100 $\mu$m, 30 $\mu$m to 100 $\mu$m, 35 $\mu$m to 100 $\mu$m, 40 $\mu$m to 100 $\mu$m, 50 $\mu$m to 100 $\mu$m, or 60 $\mu$m to 100 $\mu$m, preferably 30 $\mu$m to 100 $\mu$m.

In this specification, the average pore diameter on the surface of the porous polymer film is the area average pore diameter. The area average pore diameter can be determined according to the following (1) and (2). Incidentally, the average pore diameter of the portion other than the surface of the porous polymer film can be similarly determined.

(1) From the scanning electron micrograph of the surface of the porous film, the pore area S is measured for 200 or more open pore portions, and each pore diameter d is calculated from the following Equation I assuming the pore shape as a perfect circle.

[Math. 1]

$$\text{Pore Diameter } d = 2 \times \sqrt{(S/\pi)} \qquad \text{Equation I}$$

(2) All the pore diameters obtained by the above Equation I are applied to the following Equation II to determine the area average pore diameter da when the shape of the pores is a perfect circle.

[Math. 2]

$$\text{Area Average pore Diameter } da = \sum (d^3) / \sum (d^2) \qquad \text{Equation II}$$

The thicknesses of the surface layers A and B are not particularly limited, but is, for example, 0.01 to 50 $\mu$m, preferably 0.01 to 20 $\mu$m.

The average pore diameter of macrovoids in the planar direction of the film in the macrovoid layer in the porous polymer film is not particularly limited but is, for example, 10 to 500 $\mu$m, preferably 10 to 100 $\mu$m, and more preferably 10 to 80 $\mu$m. The thicknesses of the partition wall in the macrovoid layer are not particularly limited, but is, for example, 0.01 to 50 $\mu$m, preferably 0.01 to 20 $\mu$m. In an embodiment, at least one partition wall in the macrovoid layer has one or two or more pores connecting the neighboring macrovoids and having the average pore diameter of 0.01 to 100 $\mu$m, preferably 0.01 to 50 $\mu$m. In another embodiment, the partition wall in the macrovoid layer has no pore.

The total film thickness of the porous polymer film used for the invention is not particularly limited, but may be 5 $\mu$m or more, 10 $\mu$m or more, 20 $\mu$m or more or 25 $\mu$m or more, and 500 $\mu$m or less, 300 $\mu$m or less, 100 $\mu$m or less, 75 $\mu$m or less, or 50 $\mu$m or less. It is preferably 5 to 500 $\mu$m, and more preferably 25 to 75 $\mu$m.

The film thickness of the porous polymer film used for the invention can be measured using a contact thickness gauge.

The porosity of the porous polymer film used in the present invention is not particularly limited but is, for example, 40% or more and less than 95%.

The porosity of the porous polymer film used for the invention can be determined by measuring the film thickness and mass of the porous film cut out to a prescribed size, and performing calculation from the basis weight according to the following Equation III.

[Math. 3]

$$\text{Porosity } \% = (1 - w / (S \times d \times D)) \times 100 \quad \text{Equation III}$$

(wherein S represents the area of the porous film, d represents the total film thickness, w represents the measured mass, and D represents the polymer density. The density is defined as 1.34 g/cm$^3$ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 $\mu$m to 25 $\mu$m, and the average pore diameter of the pore present on the surface layer B is 30 $\mu$m to 100 $\mu$m; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 $\mu$m; wherein the pores on the surface layers A and B communicate with the macrovoid, the total film thickness is 5 to 500 $\mu$m, and the porosity is 40% or more and less than 95%. In an embodiment, at least one partition wall in the macrovoide layer has one or two or more pores connecting the neighboring macrovoids with each other and having the average pore diameter of 0.01 to 100 $\mu$m, preferably 0.01 to 50 $\mu$m. In another embodiment, the partition wall does not have such pores.

The porous polymer film used for the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the present invention is not particularly limited so long as it has the structural features described above and includes, preferably a porous polyimide film or porous polyethersulfone film (PES).

< Porous polyimide film >

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide film usable for the present invention is a porous polyimide film preferably containing polyimide (as a main component) obtained from tetracarboxylic dianhydride and diamine, more preferably a porous polyimide film composed of tetracarboxylic dianhydride and diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

In an embodiment, the porous polyimide film usable for the present invention includes a colored porous polyimide film obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which may be used for the invention is preferably a porous polyimide film comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units, and/or,
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used in the present invention is preferably a three-layer structure porous polyimide film having a surface layer A and a surface layer B, the surface layers having a plurality of pores, and a macrovoid layer sandwiched between the surface layers A and B; wherein an average pore diameter of the pores present in the surface layer A is 0.01 $\mu$m to 25 $\mu$m, and the mean pore diameter present on the surface layer B is 30 $\mu$m to 100 $\mu$m; wherein the macrovoid layer has a partition wall bonded to the surface layers A and B, and a plurality of macrovoids surrounded by such a partition wall and the surface layers A and B; wherein the thickness of the macrovoid layer, and the surface layers A and B is 0.01 to 20 $\mu$m, wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide films described in WO2010/038873, Japanese Unexamined Patent Publication (Kokai) No. 2011-219585 or Japanese Unexamined Patent Publication (Kokai) No. 2011-219586 may be used for the present invention.

< Porous Polyethersulfone Film (Porous PES Film) >

The porous polyethersulfone film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone membrane; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous polyethersulfone film or polyethersulfone as a raw material thereof has a glass transition temperature of 200°C or higher, or that a distinct glass transition temperature is not observed.

The method for producing the porous polyethersulfone film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and

a step in which the coagulated film having pores obtained in the above-mentioned step is heat-treated for coarsening of the aforementioned pores to obtain a porous polyethersulfone film;

wherein the heat treatment includes the temperature of the coagulated film having the pores is raised higher than the glass transition temperature of the polyethersulfone, or up to 240°C or higher.

The porous polyethersulfone film which can be used in the present invention is preferably a porous polyethersulfone film having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;

wherein the thickness of the macrovoid layer is 0.1 to 50 $\mu$m,

each of the surface layers A and B has a thickness of 0.1 to 50 $\mu$m,

wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,

wherein one of the surface layers A and B has a surface aperture ratio of 15% or more while other has a surface aperture ratio of 10% or more,

wherein the pores of the surface layers A and B communicate with the macrovoids,

wherein the porous polyethersulfone film has total film thickness of 5 to 500 $\mu$m and a porosity of 50 to 95%.

<< Cell Culture Module >>

In this specification, a "cell culture module" refers to a cell culture substrate applicable to a cell culture vessel and a cell culture device.

A cell culture vessel and a cell culture device in which the cell culture module of the present invention can be used are not particularly limited, but, for example, the cell culture module can be used in a cell culture vessel and a cell culture device which are commercially available. For example, the cell culture module can be used in a culture device including a culture vessel composed of a flexible bag, and can be used in the state of floating in the culture vessel. In addition, for example, it is possible to apply the cell culture module to a stirring culture type vessel such as a spinner flask, and to culture cells. In addition, as for a culture vessel, it may be applicable to an open vessel and a closed vessel. For example, any of a petri dish, a flask, plastic bag, a test tube, and a large tank for cell culture may be used, as appropriate. These include, for example, Cell Culture Dish manufactured by BD Falcon, and Nunc Cell Factory manufactured by Thermo Scientific. A sterilized bottle, for example, a simple columnar vessel such as a storage bottle manufactured by Corning, Inc. can also be efficiently used as a culture vessel by using a shaking device such as an orbital shaker or a program shaker. Similarly, use of a shaker enables a petri dish or a flask to be used as a culture vessel.

It is preferred that the configuration member of the cell culture module of the present invention, except the porous polymer films, has enough strength not to be deformed by movement of the culture medium under stirring culture, shaking culture conditions, and that the member is formed of a non-flexible material. Moreover, it is preferred that the configuration member is formed of a material which does not affect the growth of cells in cell culture. Examples of such materials include, for example, polymers such as polyolefins (for example, polyethylene and polypropylene), nylon, polyester, polystyrene, polycarbonate, polymethyl methacrylate, and polyethylene terephthalate; and metals such as stainless steel and titanium, but are not limited thereto.

< Cell Culture Module A >

One mode of the cell culture module of the present invention is a cell culture module including:

an apex part;
a bottom part;
a side part including a culture medium flow inlet;
a plurality of partition parts that partition a space formed by the apex part, the bottom part, and the side part, and include a culture medium flow inlet; and
a porous polymer film fixed to each of two or more gap spaces selected from a gap space between the apex part and a partition part adjacent thereto, a gap space between the bottom part and a partition part adjacent thereto, and a plurality of gap spaces between partition parts adjacent to each other,
wherein the porous polymer film is a porous polymer film with a three-layer structure, including a surface layer A and a surface layer B including a plurality of pores, as well as a macrovoid layer sandwiched between the surface layer A and the surface layer B, the average pore diameter of the pores present in the surface layer A is smaller than the average pore diameter of the pores present in the surface layer B, the macrovoid layer includes a partition wall bonded to the surface layers A and B, and a plurality of macrovoids surrounded by the partition wall and the surface layers A and B, and the pores in the surface layers A and B communicate with the macrovoids.

Hereinafter, the cell culture module also refers to "cell culture module A".
A "partition part" in the cell culture module of the present invention is generally planar. An "apex part" in the cell culture module of the present invention is a part present in the uppermost part generally vertical to the extending direction of the partition part, and the shape of the apex part is not particularly limited. A "bottom part" in the cell culture module of the present invention is a part present in the lowermost part generally vertical to the extending direction of the partition part, and the shape of the bottom part is not particularly limited. In the cell culture module of the present invention, a "side part" is a portion, other than the apex part and the bottom part, configuring the periphery of the cell culture module, and the shape of the side part is not particularly limited.
The apex and bottom parts of the cell culture module A may include culture medium flow inlets. The numbers and shapes of the culture medium flow inlets present in the apex and bottom parts are not particularly limited as long as a culture medium is favorably supplied into the interior of the module. Moreover, the sizes thereof are not particularly limited unless cultured cells and the culture medium are prevented from passing. Preferably, each of the apex and bottom parts includes a plurality of culture medium flow inlets.
The side part of the cell culture module A includes culture medium flow inlets. The number and shapes of the culture medium flow inlets present in the side part are not particularly limited as long as a culture medium is favorably supplied into the interior of the module. Moreover, the sizes thereof are not particularly limited unless cultured cells and the culture medium are prevented from passing. Preferably, the side part includes a plurality of culture medium flow inlets.
The number of the partition parts of the cell culture module A is not particularly limited, but is preferably 2 to 50, more preferably 2 to 30, still more preferably 2 to 20, and particularly preferably 2 to 10. The number and shapes of the culture medium flow inlets included in the partition part are not particularly limited unless a culture medium is prevented from favorably moving. Moreover, the sizes thereof are not particularly limited unless cultured cells and the culture medium are prevented from passing.
A method of fixing a porous polymer film to a gap space is not particularly limited. For example, the porous polymer film is fixed by being sandwiched between an apex part and a partition part adjacent thereto, between a bottom part and a partition part adjacent thereto, and between partition parts adjacent to each other. For example, at least one place of the porous polymer film is fixed to at least one place of the apex, bottom, or partition part forming the gap space by an optional method (for example, adhesion with an adhesive, or fixation using a fastener). The fixation of the porous polymer film to the gap space can prevent stress from being applied to cells to be grown in the porous polymer film, resulting in suppression of apoptosis or the like, enabling stable culture of a large amount of cells.
The porous polymer film can be fixed in an optional form to the gap space. In one embodiment, the porous polymer film is a layered body of a plurality of porous polymer films. The layered porous polymer films may be small pieces. The shape of the small pieces may be an optional shape such as, for example, a circular, elliptical, quadrangular, triangular, polygonal, or string shape. Preferably, the small pieces of the layered porous polymer films have a generally square shape. The size of the small pieces may be an optional size. When the small pieces have a generally square shape, the length thereof is not particularly limited but is, for example, 80 mm or less, 50 mm or less, 30 mm or less, 20 mm or less, or 10 mm or less.
When the porous polymer film fixed to the gap space is a layered body of the plural porous polymer films, the layered body is preferably a layered body of two or more, three or more, four or more, or five or more, and 100 or less, 50 or less, 40 or less, 30 or less, 20 or less, 15 or less, or 10 or less porous polymer films, more preferably a layered body of 3 to 100 porous polymer films, and more preferably a layered body of 5 to 50 porous polymer films.

When the porous polymer film fixed to the gap space is a layered body of the plural porous polymer films, insoles may be disposed between the porous polymer films. A culture medium can be efficiently supplied to between the layered porous polymer films by disposing the insoles. The insoles are not particularly limited as long as having the function of forming optional spaces between the layered porous polymer films and efficiently supplying a medium. For example, a planar structure having a mesh structure can be used as such an insole. For example, a mesh made of polystyrene, polycarbonate, polymethyl methacrylate, polyethylene terephthalate, or stainless steel can be used as the material of the insoles. However, the material is not limited thereto. When the insoles having a mesh structure are possessed, openings to such a degree that a culture medium can be supplied to between the layered porous polymer films may be included, and can be selected if appropriate.

In one embodiment, porous polymer films fixed to gap spaces may be processed into a three-dimensional shape rather than a planar shape, and used. For example, the porous polymer films, i) which are folded up, ii) which are wound into a roll-like shape, iii) of which the sheets or small pieces are linked in a thready structure, or iv) which are tied into a rope-like shape, may be used.

The entire shape of the cell culture module A is not particularly limited. Cell culture modules having various entire shapes can be produced by changing the shapes of members configuring apex, bottom, side, and partition parts. From the viewpoint of the easiness of stirring in culture, the easiness of production, or the like, the entire shape of the cell culture module A is preferably an n-prism shape (for example, n = 3 to 6), a cylindrical shape, a spheroid shape, an ovoid shape, or a spherical shape, and more preferably a rectangular parallelepiped shape, a cube shape, or an ovoid shape. When cell culture modules have an n-prism shape or a cylindrical shape, the corners of the modules may be chamfered in order to lessen impact at the time of the collision between the modules and to avoid damage to the modules.

Preferably, at least one of gap spaces adjacent to a gap space to which a porous polymer film is fixed does not include a porous polymer film. For example, when a porous polymer film is fixed to a gap space between an apex part a partition part adjacent thereto, there is only one adjacent gap space, and therefore, the space does not include a porous polymer film. For example, when a porous polymer film is fixed to a gap space between a bottom part and a partition part adjacent thereto, there is only one adjacent gap space, and therefore, the space does not include a porous polymer film. For example, when a porous polymer film is fixed to a gap space between partition parts adjacent to each other, there are two gap spaces adjacent to each other, and therefore, neither thereof includes a porous polymer film or either thereof does not include a porous polymer film. A gap space including no porous polymer film functions as a clearance for passage of a culture medium. A culture medium is supplied from a culture medium flow inlet to a clearance for passage of a culture medium, present in the interior of the cell culture module A. The culture medium supplied to the clearance for passage of a culture medium can pass through a culture medium flow inlet present in a partition part and can efficiently come into contact with a porous polymer film.

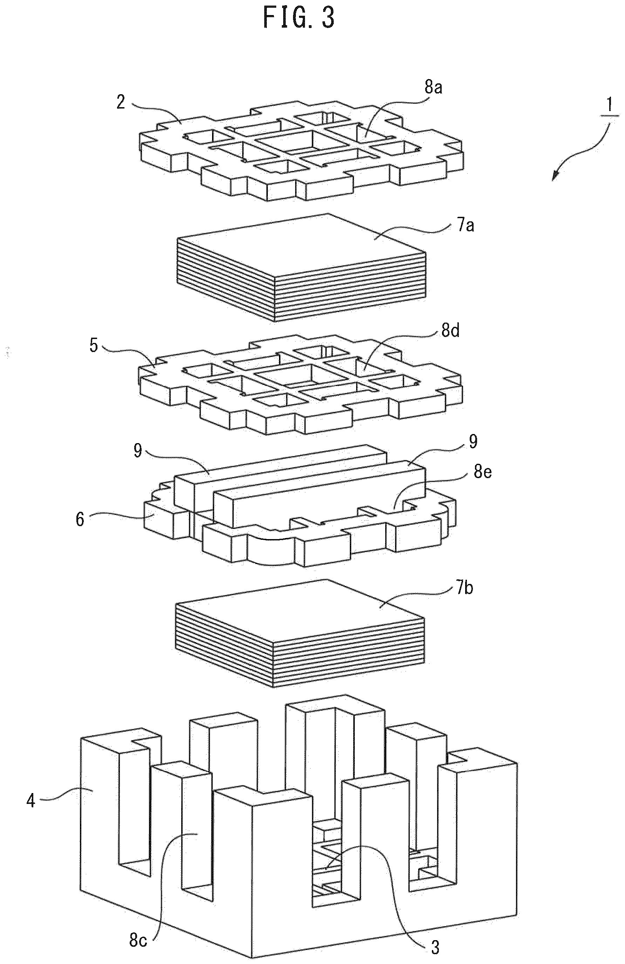

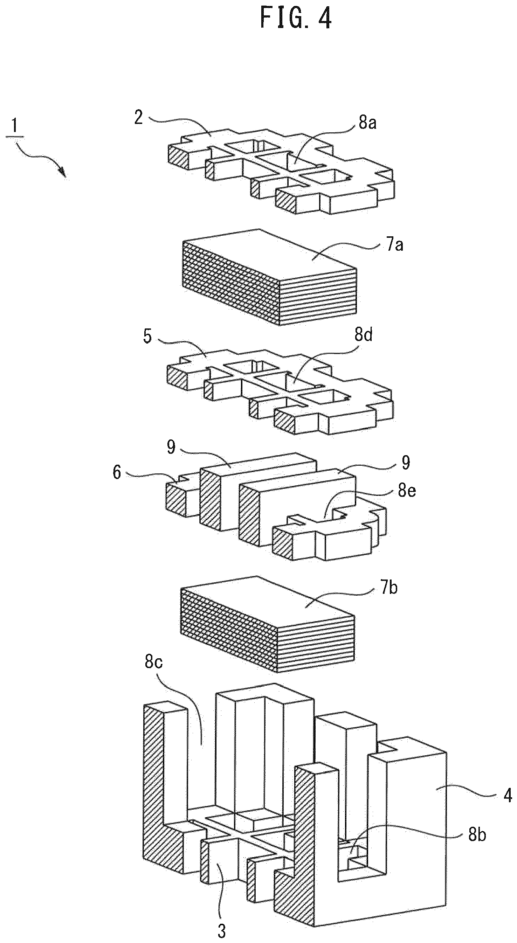

The cell culture module A may include arrangement means for arranging the apex part, the partition part, and the bottom part at regular spacings in order to dispose gap spaces between the apex part and the partition part adjacent thereto, between the bottom part and the partition part adjacent thereto, and between the partition parts adjacent to each other. The shape of the arrangement means is not particularly limited, but is determined depending on the configuration of the cell culture module, as appropriate. For example, a mount stage of partition parts adjacent to each other, disposed on a partition part, as illustrated in FIGs. 3 and 4, is acceptable. For example, a mount stage for mounting the partition part and the apex part, disposed on the inner wall of the side part of the vessel formed by the bottom part and the side part, is also acceptable. For example, a (for example, columnar) linking member for linking the members configuring the apex part, the bottom part, and the partition part to each other is also acceptable.

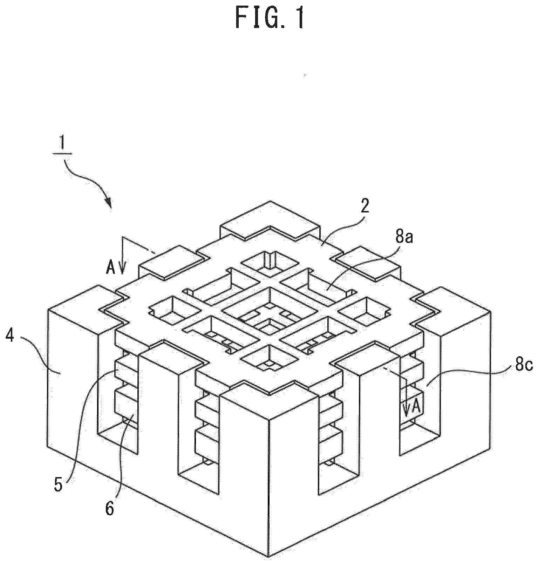

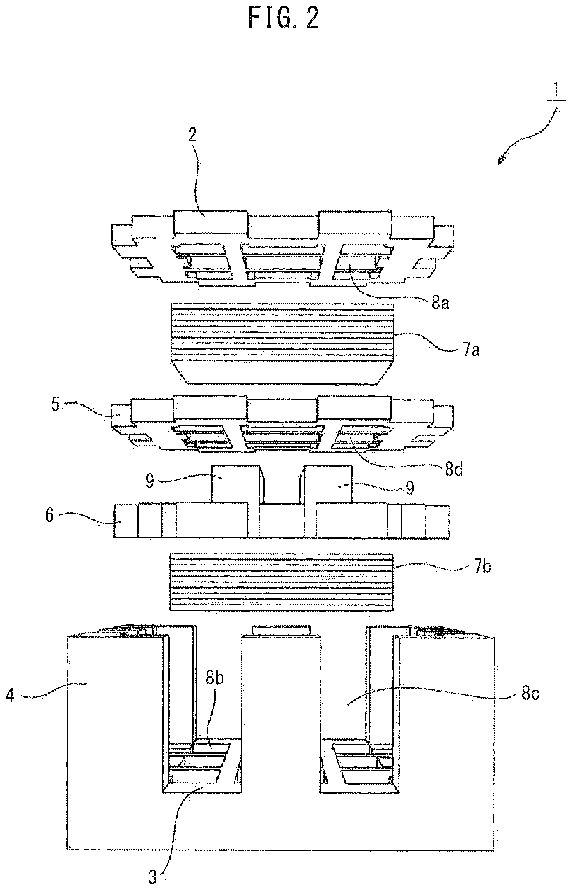

FIGs. 1 to 4 illustrate a configuration example of the cell culture module A. A cell culture module 1 includes an apex part 2, a bottom part 3, a side part 4, a first partition part 5, and a second partition part 6. Moreover, porous polymer film layered bodies 7a and 7b are sandwiched between the apex part 2 and the first partition part 5, and between the bottom part 3 and the second partition part 6, of the cell culture module 1. The apex part 2, bottom part 3, side part 4, first partition part 5, and second partition part 6 of the cell culture module 1 include culture medium flow inlets 8a to 8e, respectively.

Mount stages 9 for mounting the adjacent first partition part 5 are disposed on the top surface of the second partition part 6. The mount stages 9 function as arrangement means for arranging the first partition part 5 and the second partition part 6 at a regular spacing. The mount stages 9 allow a gap space in which a porous polymer film is not placed to be formed between the second partition part 6 and the first partition part 5. The gap space functions as a clearance for passage of a culture medium. A culture medium supplied from a culture medium flow inlet present in each of the apex part 2, the bottom part 3, and the side part 4 to the clearance for passage of a culture medium can pass through the culture medium flow inlet 8 present in each of the first partition part 5 and the second partition part 6, and can efficiently come into contact with the porous polymer film layered bodies 7a and 7b. The number, positions, and shapes of the mount stages are not particularly limited. The mount stages may be disposed on the undersurface of the adjacent first partition part 5 rather than on the top surface of the second partition part 6. Moreover, the mount stages may be disposed on the inner wall surface of the side part of the vessel, formed by the bottom part 3 and the side part 4.

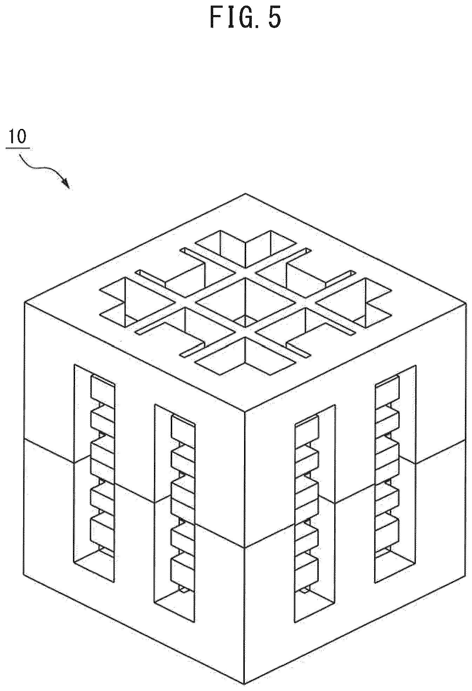

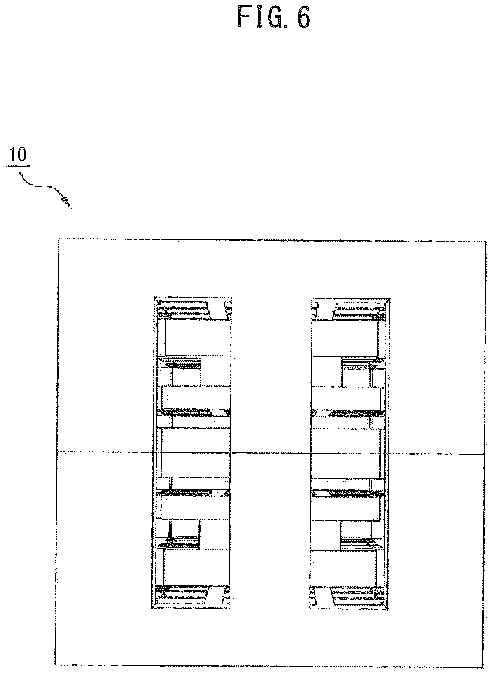

When the cell culture module A has a rectangular parallelepiped shape or a cube shape, a plurality of cell culture modules may be linked, resulting in formation of a cell culture module complex. FIGs. 5 and 6 illustrate a configuration example of a cell culture module complex in which two cell culture modules having a rectangular parallelepiped shape are linked. The cell culture module complex 10 in FIGs. 5 and 6 is produced by preparing the two cell culture modules 1 illustrated in FIGs. 1 to 4, and linking the apex parts of one cell culture module and the other cell culture module to each other, and has a cube shape. The cell culture module complex in FIGs. 5 and 6 can be regarded as a cell culture module with a cube shape, including:

an apex part having a culture medium flow inlet;
a bottom part having a culture medium flow inlet;
a side part having a culture medium flow inlet;
five partition parts that partition a space formed by the apex part, the bottom part, and the side part, and have culture medium flow inlets; and
a porous polymer film fixed to each of four gap spaces selected from a gap space between the apex part and a partition part adjacent thereto, a gap space between the bottom part and a partition part adjacent thereto, and a plurality of gap spaces between partition parts adjacent to each other,
wherein at least one of the gap spaces adjacent to the gap space to which the porous polymer film is fixed does not include a porous polymer film.

Accordingly, the cell culture module complex can be regarded as one embodiment of the cell culture module A.

< Cell Culture Module B >

A cell culture module including:

a plurality of cell culture submodules; and
a casing for containing a cell culture submodule, which is used for layering and containing the plurality of cell culture submodules and includes a culture medium flow inlet,
wherein the cell culture submodules include:

a porous polymer film; and
a casing for containing a porous polymer film, including a culture medium flow inlet, wherein the porous polymer film is fixed and contained, and
wherein the porous polymer film is a porous polymer film with a three-layer structure, including a surface layer A and a surface layer B including a plurality of pores, as well as a macrovoid layer sandwiched between the surface layer A and the surface layer B, the average pore diameter of the pores present in the surface layer A is smaller than the average pore diameter of the pores present in the surface layer B, the macrovoid layer includes a partition wall bonded to the surface layers A and B, and a plurality of macrovoids surrounded by the partition wall and the surface layers A and B, and the pores in the surface layers A and B communicate with the macrovoids.

Hereinafter, the cell culture module is also referred to as a "cell culture module B".

The shape of the membrane-like porous polymer film can be prevented from being continuously deformed because a porous polymer film is fixed and contained in a casing. This can prevent stress from being applied to cells to be grown in the porous polymer film, resulting in suppression of apoptosis or the like, enabling stable culture of a large amount of cells. A method of fixing a porous polymer film into a casing for containing a porous polymer film is not particularly limited. For example, the porous polymer film is fixed by being sandwiched between the apex and bottom parts of the casing. For example, at least one place of the porous polymer film is fixed to at least one place in the casing by an optional method (for example, adhesion with an adhesive, or fixation using a fastener).

The shape of the porous polymer film contained in the casing is not particularly limited. In one embodiment, the porous polymer film is a layered body of a plurality of porous polymer films. The layered porous polymer films may be small pieces. The shape of the small pieces may be an optional shape such as, for example, a circular, elliptical, quadrangular, triangular, polygonal, or string shape. Preferably, the small pieces of the layered porous polymer films have a generally square shape. The size of the small pieces may be an optional size. When the small pieces have a generally square shape, the length thereof is not particularly limited but is, for example, 80 mm or less, 50 mm or less, 30 mm or less, 20 mm or less, or 10 mm or less.

When the porous polymer film contained in the casing is a layered body of the plural porous polymer films, the layered body is preferably a layered body of two or more, three or more, four or more, or five or more, and 100 or less, 50 or less, 40 or less, 30 or less, 20 or less, 15 or less, or 10 or less porous polymer films, more preferably a layered body of 3 to 100 porous polymer films, and particularly preferably a layered body of 5 to 50 porous polymer films. In the layered body, insoles may be disposed between the porous polymer films. A culture medium can be efficiently supplied to between the layered porous polymer films by disposing the insoles. The insoles are not particularly limited as long as having the function of forming optional spaces between the layered porous polymer films and efficiently supplying a medium. For example, a planar structure having a mesh structure can be used as such an insole. For example, a mesh made of polystyrene, polycarbonate, polymethyl methacrylate, polyethylene terephthalate, or stainless steel can be used as the material of the insoles. However, the material is not limited thereto. When the insoles having a mesh structure are possessed, openings to such a degree that a culture medium can be supplied to between the layered porous polymer films may be included, and can be selected if appropriate.

In one embodiment, porous polymer films contained in a casing may be processed into a three-dimensional shape rather than a planar shape, and used. For example, the porous polymer films, i) which are folded up, ii) which are wound into a roll-like shape, iii) of which the sheets or small pieces are linked in a thready structure, or iv) which are tied into a rope-like shape, may be used.

The casing for containing a porous polymer film, which contains a porous polymer film, includes culture medium flow inlets. A cell culture medium is supplied into the interior of the casing, and discharged to the exterior thereof through the flow inlets. The number and shapes of the culture medium flow inlets are not particularly limited. Moreover, the sizes thereof are not particularly limited unless cultured cells and the culture medium are prevented from passing.

The culture medium flow inlets in the casing for containing a porous polymer film may have a mesh-like structure. Moreover, the casing itself containing a porous polymer film may have a mesh shape. Examples of the structure having a mesh shape include, but are not limited to, those having structures having longitudinal, transverse, and/or oblique grating patterns wherein each opening forms a culture medium flow inlet which allows the fluid to pass therethrough.

In the cell culture module B, a plurality of cell culture submodules are contained in a casing for containing a cell culture submodule. The number of the layered cell culture submodules is preferably 2 or more, 3 or more, 4 or more, or 5 or more, and 30 or less, 15 or less, or 10 or less, more preferably 2 to 15, particularly preferably 3 to 10.

The casing for containing a cell culture submodule includes culture medium flow inlets. A cell culture medium is supplied into the interior of the casing, and discharged to the exterior thereof through the flow inlets. The number and shapes of the culture medium flow inlets are not particularly limited. Moreover, the sizes thereof are not particularly limited unless cultured cells and the culture medium are prevented from passing. Preferably, the casing for containing a cell culture submodule includes a plurality of culture medium flow inlets.

The cell culture module B preferably includes gap spaces between the plurality of layered cell culture submodules and the casing for containing a cell culture submodule. The gap spaces function as clearances for passage of a culture medium. A culture medium is supplied from the culture medium flow inlets present in the casing for containing a cell culture submodule to the clearances for passage of a culture medium, present in the interior of the cell culture module B. The culture medium supplied to the clearances for passage of a culture medium can pass through the culture medium flow inlets present in the casing for containing a porous polymer film and can efficiently come into contact with the porous polymer films.

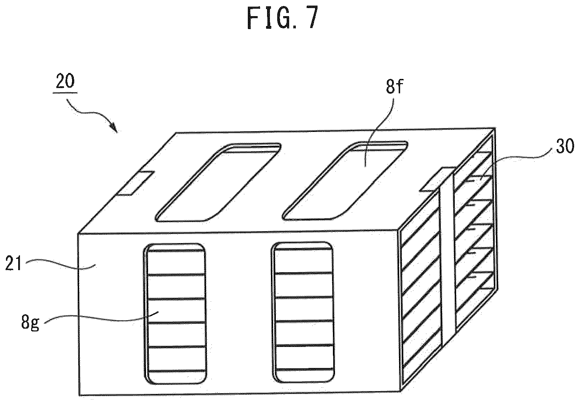

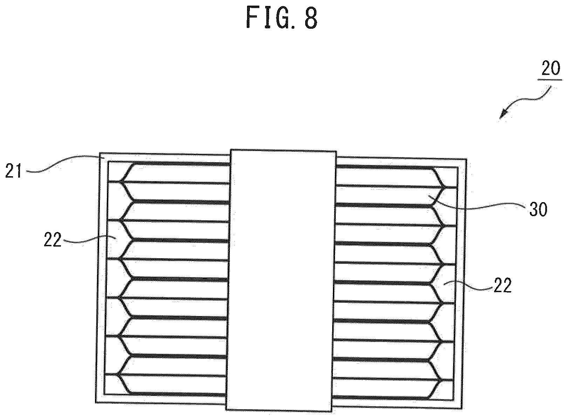

FIGs. 7 and 8 illustrate a configuration example of the cell culture module B. In a cell culture module 20, cell culture submodules 30 are layered. The layered body is contained in a casing 21 for containing a cell culture submodule. The layered body is sandwiched between the apex and bottom parts of the casing 21 for containing a cell culture submodule. The casing 21 for containing a cell culture submodule includes culture medium flow inlets 8f and 8g in the apex and side parts thereof. The casing 21 for containing a cell culture submodule also includes culture medium flow inlets in the bottom part thereof, which are not illustrated in the drawings. As illustrated in FIG. 8, gap spaces 22 are present between the plurality of layered cell culture submodules 30 and the casing 21 for containing a cell culture submodule. The gap spaces function as clearances for passage of a culture medium.

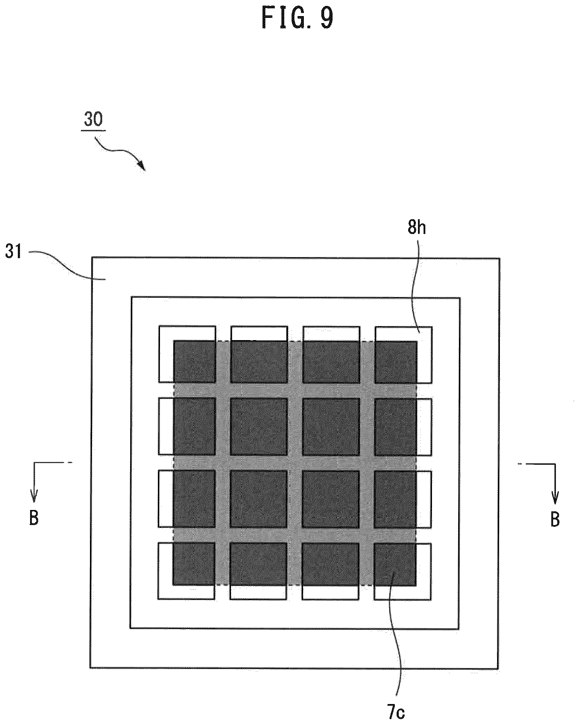

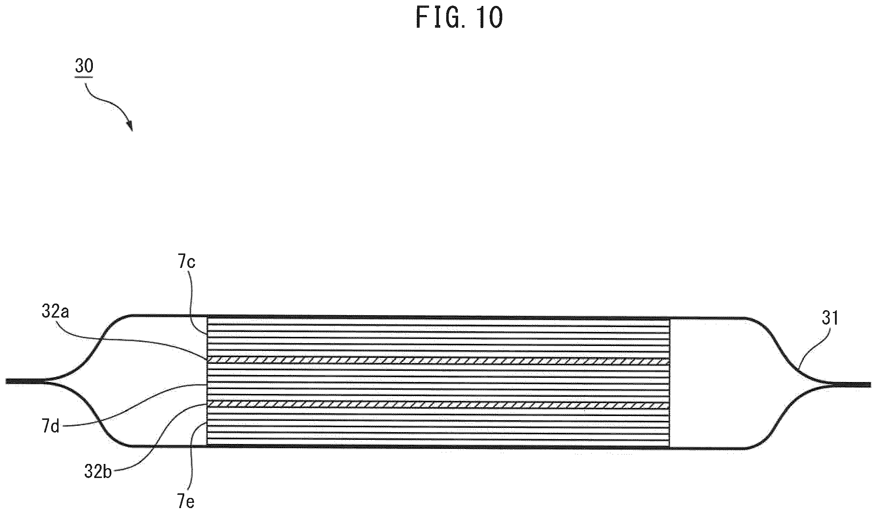

FIGs. 9 and 10 illustrate a configuration example of the cell culture submodules which are the components of the cell culture module 20 illustrated in FIGs. 7 and 8. A set of six porous polymer film layered bodies 7c, a set of six porous polymer film layered bodies 7d, and a set of six porous polymer film layered bodies 7e are included. In a casing 31 for containing a porous polymer film. insoles 32a and 32b are contained between the porous polymer film layered bodies 7c and 7d, and between the porous polymer film layered bodies 7d and 7e, respectively. The top surface of the casing 31 for containing a porous polymer film includes a plurality of culture medium flow inlets 8h. The undersurface thereof similarly includes culture medium flow inlets. The cell culture submodules illustrated in FIGs. 9 and 10 can be produced by sandwiching a layered body of polymeric films between two flat substrates, and then fixing the peripheral edges of the flat substrates.

The cell culture module of the present invention illustrated in FIGs. 1 to 8 has an excellent liquid flow property into the interior of the module, and enables oxygen and a nutrient to be favorably supplied to the porous polymer films contained in the module. Moreover, the entire module has a high floating force, and the plurality of modules can be efficiently stirred. Moreover, a homogeneous culture condition can be applied to the porous polymer films in the module. Further, polymer porosity is fixed, and therefore, stress can be prevented from being applied to cells to be grown in the porous polymer films, resulting in suppression of apoptosis or the like, enabling stable culture of a large amount of cells. Moreover, the cell culture module of the present invention has high strength, and is advantageous for long-term cell culture and the long-term continuous production of a substance with cultured cells.

All documents mentioned in this specification are incorporated herein by reference in their entirety.

Examples of the present invention described below are only for illustration, and are not intended to limit the technical scope of the present invention. The technical scope of the present invention is limited only by the descriptions of claims. Change of the present invention, for example, addition, deletion, and substitution of a constituent feature of the present invention can be made without departing from the gist of the present invention.

EXAMPLES

The porous polyimide film used in the following examples and comparative examples was prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide film was a three-layer structure porous polyimide film having a surface layer A and a surface layer B, the surface layers having a plurality of pores, and a macrovoid layer sandwiched between the surface layers A and B; wherein the average pore diameter of the pore present on the surface layer A was 19 $\mu$m, the average pore diameter of the pore present on the surface layer B was 42 $\mu$m, and the film thickness was 25 $\mu$m, and the porosity was 74%.

Example 1

Cell Culture Using Cell Culture Module Complex 10

Cell culture was performed using a cell culture module complex 10 illustrated in FIGs. 5 and 6. Two cell culture modules 1 illustrated in FIGs. 1 to 4 were prepared, the apex parts thereof were allowed to face each other, and were linked by being thermally fused by a soldering iron, to prepare the cell culture module complex 10 having a cube shape. The size of each porous polyimide film used in the present example is 1.4 cm × 1.4 cm. The cell culture module complex 10 includes four layered bodies of which each set includes ten porous polyimide films, and the total area thereof is 80 cm$^2$. The cell culture module complex 10 was washed with a diluted Milton solution (manufactured by KYORIN Pharmaceutical Co., Ltd.), ultrapure water, and 70% ethanol-containing water, dried in a sterilization manner, and then used.

Conditioned/suspended anti-human IL-8 antibody producing CHO-DP12 cells (ATCC CRL-12445) were float-cultured in a medium (BalanCD (Trademark) CHO Growth A) and culture was continued until viable cell count per mL was 3.51 × 10$^6$ cells/mL (total cell number of 3.83 × 10$^6$ cells/mL, viable cell rate of 92%).

The three cell culture module complexes 10 were added into a bottle type sterilized vessel (manufactured by Corning, Inc.) having an internal volume of 150 mL, 53.3 mL of a medium for culturing a CHO cell monolayer KBM270 (manufactured by Kohjin Bio Co., Ltd.) was added into the bottle type sterilized vessel, and the three cell culture module complexes 10 were immersed in the medium at a shaking rate of 35rpm for 10 minutes in a CO$_2$ incubator using a program shaker (manufactured by KENIS LIMITED). A liquid mixture of 4.0 mL of CHO DP-12 floating cell culture medium (total cell number of 3.83 × 10$^6$ cells/mL, viable cell count of 3.51 × 10$^6$ cells/mL, dead cell count of 3.23 × 10$^5$ cells/mL, and viable cell rate of 92%) and 22.6 mL of medium for floating cells (BalanCD (Trademark) CHO Growth A) was added, and cells were adsorbed for about 14 hours using a program shaker (manufactured by KENIS LIMITED) under a rotational condition of 35 rpm (expected average viable cell adsorption number of 5.30 × 10$^4$ cells per sheet). The cell adsorption ratio calculated from the collected medium was 95%.

Then, the medium was removed, 40 mL of a medium for culturing a CHO cell monolayer KBM270 (manufactured by Kohjin Bio Co., Ltd.) was added, and culture was started. Three days after the start of the culture, the vessel was transferred to an electromagnetic orbital shaker (for a CO$_2$ incubator, manufactured by AS ONE Corporation), and the culture was continued at a shaking rate of 200 rpm. Medium replacement was performed every day, and the amounts of consumed glucose, produced lactic acid, lactate dehydrogenase, and produced antibody per day in the medium were measured using Cedex Bio (manufactured by Roche Diagnostics K.K.). It was confirmed that glucose was consumed with time, and an antibody and lactic acid were continuously produced. The amounts of consumed glucose and produced lactic acid for 3 days after the start of the culture are set forth in Table 1. It was confirmed that the culture was stably performed.

[Table 1]

Table 1

| Day | Amount of Consumed Glucose mg/L | Amount of Produced Lactic Acid mg/L |
|---|---|---|
| Day 1 | 110 | 135 |
| Day 2 | 287 | 170 |
| Day 3 | 300 | 134 |

Example 2

Cell Culture Using Cell Culture Module 20

Cell culture was performed using a cell culture module 20 illustrated in FIGs. 7 and 8. The cell culture module 20 was washed with a diluted Milton solution (manufactured by KYORIN Pharmaceutical Co., Ltd.), ultrapure water, and 70% ethanol-containing water, dried in a sterilization manner, and then used. The size of each porous polyimide film used in the cell culture module 20 used in the present example is 1.0 cm $\times$ 1.0 cm. Three layered bodies of which each set includes the six porous polyimide films were prepared, and insoles were sandwiched between the layered bodies. The total number of the porous polyimide films in the cell culture module 20 used in the present example is 108, and the total area thereof is 108 cm$^2$. Since the five cell culture modules 20 were used in the present example, the total number of the porous polyimide films is 540, and the total area thereof is 540 cm$^2$. Each of the materials of the casing for containing a cell culture submodule and the casing for containing a porous polymer film of the cell culture modules 20 is a polyolefin resin. Moreover, the insoles used are meshes manufactured by NBC Meshtec Inc.

Conditioned/suspended anti-human IL-8 antibody producing CHO-DP12 cells (ATCC CRL-12445) were float-cultured using a medium (BalanCD (Trademark) CHO Growth A) and culture was continued until viable cell count per mL was $5.41 \times 10^6$ cells/mL (total cell number of $6.16 \times 10^6$ cells/mL, viable cell rate of 88%).

The five sterilized cell culture modules 20 were added into a bottle type sterilized vessel (manufactured by Corning, Inc.) having an internal volume of 150 mL, 26.7 mL of a medium for culturing a CHO cell monolayer KBM270 (manufactured by Kohjin Bio Co., Ltd.) was added into the bottle type sterilized vessel, and the five sterilized cell culture modules 20 were immersed in the medium at a shaking rate of 35 rpm for 10 minutes in a CO$_2$ incubator using a program shaker (manufactured by KENIS LIMITED). Then, a liquid mixture of 6.0 mL of CHO DP-12 floating cell culture medium (total cell number of $6.16 \times 10^6$ cells/mL, viable cell count of $5.41 \times 10^6$ cells/mL, dead cell count of $7.51 \times 10^5$ cells/mL, and viable cell rate of 88%) and 7.3 mL of medium for floating cells (BalanCD (Trademark) CHO Growth A) was added, and cells were adsorbed for about 14 hours using a program shaker (manufactured by KENIS LIMITED) under a rotational condition of 35 rpm (cell adsorption number of $5.30 \times 10^4$ cells per sheet). The cell adsorption ratio calculated from the collected medium was 88%.

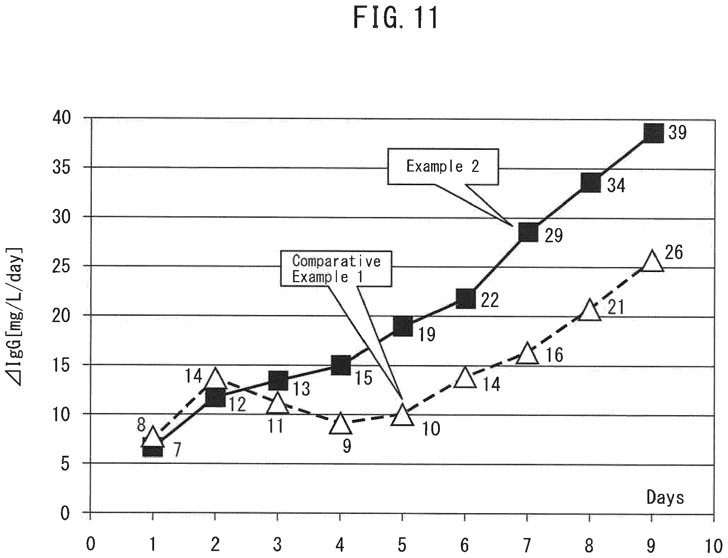

Then, the medium was removed, 40 mL of a medium for culturing a CHO cell monolayer KBM270 (manufactured by Kohjin Bio Co., Ltd.) was added, and culture was started. Two days after the start of the culture, the vessel was transferred to an electromagnetic orbital shaker (for a CO$_2$ incubator, manufactured by AS ONE Corporation), and the culture was continued at a shaking rate of 200 rpm. Medium replacement was performed every day, and the amounts of consumed glucose, produced lactic acid, lactate dehydrogenase, and produced antibody per day in the medium were measured using Cedex Bio (manufactured by Roche Diagnostics K.K.). It was confirmed that glucose was consumed with time, and an antibody and lactic acid were continuously produced. Variations with time of the amount of produced antibody are illustrated in FIG. 11.

Comparative Example 1

Thirty cell culture submodules 30 illustrated in FIGs. 9 and 10 were prepared and sterilized. The total number of porous polyimide films is 540, and the total area thereof is 540 cm$^2$. Into 150 mL storage bottles (manufactured by Corning, Inc.) filled with the submodules, 26.7 mL of a medium for culturing a CHO cell monolayer KBM270 (manufactured by Kohjin Bio Co., Ltd.) was added, and the submodules were immersed in the medium under a rotational condition of 35 rpm for 10 minutes in a CO$_2$ incubator using a program shaker (manufactured by KENIS LIMITED). A liquid mixture of 6.0 mL of CHO DP-12 floating cell culture medium (total cell number of $6.16 \times 10^6$ cells/mL, viable cell count of $5.41 \times 10^6$ cells/mL, dead cell count of $7.51 \times 10^5$ cells/mL, and viable cell rate of 88%) and 7.3 mL of medium for floating cells (BalanCD (Trademark) CHO Growth A) was added, and cells were adsorbed for about 14 hours under a rotational condition of 35 rpm (cell adsorption number of $5.83 \times 10^4$ cells per sheet). The cell adsorption ratio calculated from a cell count found from the collected medium was 97%. Medium replacement was performed every day, and the amounts of consumed glucose, produced lactic acid, lactate dehydrogenase, and produced antibody per day in the medium were measured using Cedex Bio (manufactured by Roche Diagnostics K.K.) (FIG. 11). The amount of produced antibody in the case of using the cell culture modules (Example 2) was higher.

Comparative Example 2

Conditioned/suspended anti-human IL-8 antibody producing CHO-DP12 cells (ATCC CRL-12445) were float-cultured using a medium (BalanCD (Trademark) CHO GROWTH A) and culture was continued until viable cell count per mL was $2.0 \times 10^6$ cells/mL.

Porous polyimide films having a long and narrow shape of 0.3 cm $\times$ 2.5 cm were prepared and subjected to dry heat sterilization. Then, the 11 to 12 porous polyimide films were placed in a petri dish of 20 cm$^2$, 4 mL of the floating culture medium described above was poured, and the porous polyimide films were sufficiently moisturized with a cell suspension. Then, the petri dish was left standing in a $CO_2$ incubator. After 2 hours, the petri dish was taken out of the incubator, the cell suspension was sucked and removed, 4 mL of medium (2% FBS-supplemented IMDM) was then added, and culture was further continued in the $CO_2$ incubator. The medium was replaced at a pace of every two days.

Seven days after the start of the culture, the number of cells, cultured using CCK8 (Cell Countinig Kit 8; solution reagent manufactured by Dojindo Laboratories Co., Ltd.), on the porous polyimide films in the three petri dishes was calculated as a cell count per area. On the next day, the culture in one of the three petri dishes was further continued for 2 days on an as-is basis (hereinafter referred to as "culture 1").

In one of the remaining two petri dishes, the porous polyimide film on which the cells grew was transferred into an oxygen permeable culture bag (manufactured by NIPRO CORPORATION) along with the culture medium, and sealed aseptically with a heat sealer. Then, the cells were subjected to shaking culture for 2 days in a shaker placed in a $CO_2$ incubator set to cause 20 to 30 vibrations per minute (hereinafter referred to as "culture 2").

Further, the porous polyimide film contained in the remaining petri dish was aseptically cut into about 0.3 cm $\times$ 0.3 cm strips with scissors. Then, an overcoat having a size of about 1 cm $\times$ 1 cm and made of 30 # nylon mesh was prepared, the cut porous polyimide films were contained in the overcoat, and the overcoat was aseptically sealed with a heat sealer. The obtained cell culture module was transferred along with the medium into an oxygen permeable culture bag (manufactured by NIPRO CORPORATION), and the oxygen permeable culture bag was aseptically sealed with the heat sealer. Then, the cells were subjected to shaking culture for 2 days in a shaker placed in a $CO_2$ incubator set to cause 20 to 30 vibrations per minute (hereinafter referred to as "culture 3").

The densities of the cells subjected to the cultures 1 to 3 were calculated using CCK8. The results are set forth in Table 2. In the culture 2 in which the shaking culture was performed without fixing the porous polyimide film, a marked decrease in cell count was observed. This is considered to be caused by exhibition by cells due to apoptosis or the like, resulting from stress applied to the cells growing in the porous polyimide film because of the continuous deformation of the shape of the porous polyimide film. In contrast, the decrease in cell count observed in the culture 2 was suppressed in the culture 3 in which the shaking culture was performed in a state in which the porous polyimide film was contained and fixed in the overcoat.

[Table 2]

Table 2

| | Culture 1 (cells/cm$^2$) | Culture 2 (cells/cm$^2$) | Culture 3 (cells/cm$^2$) |
|---|---|---|---|
| Before Performance | $6.6 \times 10^5$ | $6.6 \times 10^5$ | $6.9 \times 10^5$ |
| After Performance | $7.0 \times 10^5$ | $1.2 \times 10^4$ | $6.6 \times 10^5$ |
| After Performance/Before Performance | 1.06 | 0.02 | 0.72 |

INDUSTRIAL APPLICABILITY

The cell culture module of the present invention can be preferably used for stable cell culture and substance production.

REFERENCE SIGNS LIST

| | |
|---|---|
| 1 | Cell culture module |
| 2 | Apex part |
| 3 | Bottom part |
| 4 | Side part |
| 5 | First partition part |
| 6 | Second partition part |
| 7a to 7e | Layered body of porous polymer films |
| 8a to 8h | Culture medium flow inlet |
| 9 | Mount stage |
| 10 | Cell culture module complex |
| 20 | Cell culture module |
| 21 | Casing for containing cell culture submodule |
| 22 | Gap space |
| 30 | Cell culture submodule |
| 31 | Casing for containing porous polymer film |
| 32a, 32b | Insole |

**Claims**

1. A cell culture module comprising:

   an apex part;
   a bottom part;
   a side part comprising a culture medium flow inlet;
   a plurality of partition parts that partition a space formed by the apex part, the bottom part, and the side part, and comprise a culture medium flow inlet; and
   a porous polymer film fixed to each of two or more gap spaces selected from a gap space between the apex part and a partition part adjacent thereto, a gap space between the bottom part and a partition part adjacent thereto, and a plurality of gap spaces between partition parts adjacent to each other,
   wherein the porous polymer film is a porous polymer film with a three-layer structure, comprising a surface layer A and a surface layer B including a plurality of pores, as well as a macrovoid layer sandwiched between the surface layer A and the surface layer B, an average pore diameter of the pores present in the surface layer A is smaller than an average pore diameter of the pores present in the surface layer B, the macrovoid layer comprises a partition wall bonded to the surface layers A and B, and a plurality of macrovoids surrounded by the partition wall and the surface layers A and B, and the pores in the surface layers A and B communicate with the macrovoids.

2. The cell culture module according to claim 1, wherein at least one of gap spaces adjacent to the gap space to which the porous polymer film is fixed does not comprise a porous polymer film.

3. The cell culture module according to claim 1 or 2, wherein 3 to 100 porous polymer films are layered and placed on each of the two or more gap spaces selected from the gap space between the apex part and the partition part adjacent thereto, the gap space between the bottom part and the partition part adjacent thereto, and the plurality of gap spaces between the partition parts adjacent to each other.

4. The cell culture module according to any one of claims 1 to 3, wherein the porous polymer film is a porous polyimide film.

5. The cell culture module according to any one of claims 1 to 3, wherein the porous polymer film is a porous polyethersulfone film.

6. The cell culture module according to any one of claims 1 to 5, wherein the apex part and the bottom part comprise a culture medium flow inlet.

7. The cell culture module according to any one of claims 1 to 6, comprising a rectangular parallelepiped shape.

8. The cell culture module according to any one of claims 1 to 6, comprising a cube shape.

9. The cell culture module according to any one of claims 1 to 6, comprising an ovoid shape.

10. A cell culture module complex, wherein the plurality of cell culture modules according to claims 7 or 8 are connected.

11. A cell culture module comprising:

a plurality of cell culture submodules; and
a casing for containing a cell culture submodule, which is used for layering and containing the plurality of cell culture submodules and comprises a culture medium flow inlet,
wherein the cell culture submodules comprise:

a porous polymer film; and
a casing for containing a porous polymer film, comprising a culture medium flow inlet, wherein the porous polymer film is fixed and contained, and
wherein the porous polymer film is a porous polymer film with a three-layer structure, comprising a surface layer A and a surface layer B including a plurality of pores, as well as a macrovoid layer sandwiched between the surface layer A and the surface layer B, an average pore diameter of the pores present in the surface layer A is smaller than an average pore diameter of the pores present in the surface layer B, the macrovoid layer comprises a partition wall bonded to the surface layers A and B, and a plurality of macrovoids surrounded by the partition wall and the surface layers A and B, and the pores in the surface layers A and B communicate with the macrovoids.

12. The cell culture module according to claim 11, further comprising a gap space between the plurality of layered cell culture submodules and the casing for containing a cell culture submodule.

13. The cell culture module according to claim 11 or 12, wherein 3 to 100 porous polymer films are layered and contained in the casing for containing a porous polymer film.

14. The cell culture module according to any one of claims 11 to 13, wherein the porous polymer film is a porous polyimide film.

15. The cell culture module according to any one of claims 11 to 13, wherein the porous polymer film is a porous polyethersulfone film.

# FIG. 1

# FIG. 2

# FIG. 3

# FIG. 4

FIG. 5

10

# FIG. 6

FIG. 7

# FIG. 8

# FIG. 9

FIG. 10

# FIG. 11

## INTERNATIONAL SEARCH REPORT

| International application No. |
| --- |
| PCT/JP2019/002375 |

**A. CLASSIFICATION OF SUBJECT MATTER**
Int.Cl. C12M3/00(2006.01)i, C12M1/00(2006.01)i, C12N5/071(2010.01)n

According to International Patent Classification (IPC) or to both national classification and IPC

**B. FIELDS SEARCHED**

Minimum documentation searched (classification system followed by classification symbols)
Int.Cl. C12M3/00, C12M1/00, C12N5/071

Documentation searched other than minimum documentation to the extent that such documents are included in the fields searched
Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)
JSTPlus/JMEDPlus/JST7580 (JDreamIII), DWPI (Derwent Innovation)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | WO 2016/121773 A1 (UBE INDUSTRIES, LTD.) 04 August 2016, claims, paragraphs [0041], [0071], [0073], [0101], [0127] & US 2018/0016547 A1 & EP 3252148 A1, claims, paragraphs [0094], [0126], [0128], [0158], [0185] | 11-15<br>1-10 |
| Y<br>A | JP 2011-519575 A (SYNEXA LIFE SCIENCES (PTY) LTD.) 14 July 2011, claims, drawings & US 2011/0124078 A1 & EP 2297293 A1 & WO 2009/136384 A2, claims, figures | 11-15<br>1-10 |

☒ Further documents are listed in the continuation of Box C.       ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 19 April 2019 (19.04.2019) | 07 May 2019 (07.05.2019) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
| --- | --- |

Form PCT/ISA/210 (second sheet) (January 2015)

**INTERNATIONAL SEARCH REPORT**

International application No.

PCT/JP2019/002375

C (Continuation).  DOCUMENTS CONSIDERED TO BE RELEVANT

| Category* | Citation of document, with indication, where appropriate, of the relevant passages | Relevant to claim No. |
|---|---|---|
| Y<br>A | JP 2012-503688 A (GAMBRO LUNDIA AB) 09 February 2012, claims, examples & US 2011/0263022 A1 & EP 2168666 A1 & WO 2010/034466 A1, claims, examples | 11-15<br>1-10 |
| Y<br>A | JP 5-41984 A (THE DOW CHEMICAL COMPANY) 23 February 1993, claims & US 5162225 A & EP 475303 A2, claims | 11-15<br>1-10 |
| Y<br>A | JP 2009-538617 A (CHABIOTECH CO., LTD.) 12 November 2009, claims & US 2009/0130754 A1 & EP 2021464 A1 & WO 2007/139357 A1, claims | 11-15<br>1-10 |
| P, Y<br>P, A | WO 2018/021358 A1 (UBE INDUSTRIES, LTD.) 01 February 2018, claims (Family: none) | 15<br>1-14 |
| A | JP 4-341176 A (KURARAY CO., LTD.) 27 November 1992, drawings (Family: none) | 1-15 |
| A | JP 6-205665 A (GERLACH, Joerg) 26 July 1994, claims & US 5516691 A & EP 590341 A2, claims | 1-15 |
| A | JP 10-257887 A (DAINIPPON PRINTING CO., LTD.) 29 September 1998, drawings (Family: none) | 1-15 |
| A | JP 2-501352 A (RESEARCH CORPORATION TECHNOLOGIES, INC.) 17 May 1990, claims & WO 1989/000188 A1 & EP 360837 A1, claims | 1-15 |
| A | US 5416022 A (CELLEX BIOSCIENCES, INC.) 16 May 1995, fig. 12 (Family: none) | 1-15 |
| A | WO 2015/012415 A1 (UBE INDUSTRIES, LTD.) 29 January 2015, claims & US 2016/0168560 A1 & EP 3026108 A1, claims | 1-15 |
| P, A | WO 2018/021367 A1 (UBE INDUSTRIES, LTD.) 01 February 2018, claims (Family: none) | 1-15 |
| P, A | WO 2018/021368 A1 (UBE INDUSTRIES, LTD.) 01 February 2018, claims (Family: none) | 1-15 |
| P, A | WO 2018/021365 A1 (UBE INDUSTRIES, LTD.) 01 February 2018, claims (Family: none) | 1-15 |
| P, A | JP 2018-14898 A (UBE INDUSTRIES, LTD.) 01 February 2018, drawings (Family: none) | 1-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003054174 A
- WO 2010038873 A
- JP 2011219585 A
- JP 2011219586 A
- WO 2015012415 A